# EUROPEAN PATENT APPLICATION

(11) **EP 2 772 475 A1**
(43) Date of publication of application: **03.09.2014**
(21) Application number: 13157468.3
(22) Date of filing: 01.03.2013
(51) Int. Cl.: C07C 2/84, C07C 5/48

(54) **Process for the manufacture of a crude alkane and/or alkene oxidation product**

(71) Applicant: Solvay SA, 1120 Bruxelles (BE)
(72) Inventor: The designation of the inventor has not yet been filed
(74) Representative: Vande Gucht, Anne

(57) **Abstract**

Process for the manufacture of a crude alkane and/or alkene oxidation product according to which a stream of alkane and/or alkene and a stream of oxygen are fed to a catalytic oxidation reaction section comprising a first reactor and at least one other reactor, all in series, in which
- the stream of alkane and/or alkene is fed into the first reactor for catalytic oxidation with an oxygen flow in order to produce a reaction gas which is then fed successively to the following reactors in series for further catalytic oxidation;
- the stream of oxygen is divided in several oxygen flows which are fed in parallel into each of the reactors in series;
- the reaction gas is cooled down between each two successive reactors and finally produces a crude alkane and/or alkene oxidation product leaving the last reactor;
- one part of said crude alkane and/or alkene oxidation product leaving the last reactor is cooled down and recycled as recycled gas to at least one of the reactors in series while the other part of said crude alkane and/or alkene oxidation product is sent to further use.

## Description

The present invention relates to a process for the manufacture of a crude alkane and/or alkene oxidation product, especially crude ethylene. Such crude ethylene can be used for the manufacture of 1,2 - dichloroethane (DCE), vinyl chloride (VC) and polyvinyl chloride (PVC).

DCE is usually prepared by oxychlorination of ethylene using hydrogen chloride (HCl) and a source of oxygen or by direct chlorination of ethylene using chlorine. The dehydrochlorination of DCE by pyrolysis thus results in the production ofVC with release of HCl. The oxychlorination and chlorination are generally carried out in parallel and the HCl produced in the pyrolysis is used in the oxychlorination.

Processes in which VC is obtained by oxychlorination of ethane and not of ethylene are also known. Such processes have not found an industrial application up till now given that as they are conducted at high temperatures, they result in a mediocre selectivity with loss of the reactants used and costs for separating and destroying the by-products.

Oxydehydrogenation (ODH) of ethane has also been considered like in WO 2008/000693, WO 2008/000702 and WO 2008/000705 where particular schemes of process have been considered in order to obtain one or two ethylene fractions of different composition submitted to chlorination/oxychlorination.

When extrapolating such ODH of ethane on an industrial scale, flammability/explosion risks in relation with the ODH reaction appear to be a major key issue which has still to be solved. In fact, the same problem occurs with most ODH reactions, like of propane to propylene, of butane to butene or of butene to butadiene.

Oxidative Coupling of Methane (OCM) to ethylene has also been widely described in literature, which ethylene could in turn be submitted to chlorination/oxychlorination. When using OCM reactions on an industrial scale, the same flammability/explosion risks exists as with the ODH reaction. As a solution to such flammability/explosion risks, US 4899003 discloses an ODH process of ethane to ethylene using several reactors over which the total oxygen flow is split. In between each two successive reactors, the gases are cooled down and water and acetic acid are removed. In order to increase the conversion of ethane, it is proposed to put the reactors in series and to increase their number. Such a process is costly in terms of energy consumption.

US 7674944 also discloses a multi-reactor solution to the same problem and besides, an alternative solution consisting in using a single reactor but with recycling of a portion of the reaction product to it. For increasing conversion and selectivity, the former solution (multiple reactors) is set forth as preferred over the latter (recycling). This document teaches to directly cool down the reaction zone(s) and not the exiting gas stream. Although such a solution is successful on a small scale, it cannot be extrapolated to an industrial scale where the residence time of the reactives in the reactor is too low.

One object of the present invention is therefore to provide an industrial process for oxidizing an alkane and/or an alkene which has the advantage of avoiding the abovementioned problems and in particular which allows getting high conversion and selectivity while avoiding the problems linked with the flammability/explosion risks. The purpose of the present invention is also to provide a way to control the reaction temperature namely: to keep it in a narrow window so that the performance of the catalyst can be optimized.

It is based on the idea of combining multi-reactors and product recycling and on using appropriate cooling and no (or as little as possible) water removal in the reaction section of the process. The Applicant has namely found that if part of the product stream leaving the last reactor is recycled as such (as a crude product i.e. before removing water therefrom) and cooled down (preferably above dew point temperature), the fact of recycling it to at least one of the reactors brings a lot of benefits from an industrial point of view:
- it further reduces the flammability risks (dilution effect)
- it limits the reaction temperature increase resulting from the exothermic reaction in the reactors (thermal ballast effect), so that each reactor can operate in the most effective catalytic temperature range in terms of conversion and selectivity
- because of the presence of water, coking and by products formation are reduced
- it allows the production of steam through waste heat recovery.

To this effect, the invention relates to a process for the manufacture of a crude alkane and/or alkene oxidation product according to which a stream of alkane and/or alkene and a stream of oxygen are fed to a catalytic oxidation reaction section comprising a first reactor and at least one other reactor, all in series, in which
- the stream of alkane and/or alkene is fed into the first reactor for catalytic oxidation with an oxygen flow in order to produce a reaction gas which is then fed successively to the following reactors in series for further catalytic oxidation;
- the stream of oxygen is divided in several oxygen flows which are fed in parallel into each of the reactors in series;
- the reaction gas is cooled down between each two successive reactors and finally produces a crude alkane and/or alkene oxidation product leaving the last reactor;
- one part of said crude alkane and/or alkene oxidation product leaving the last reactor is cooled down and recycled as recycled gas to at least one of the reactors in series while the other part of said crude alkane and/or alkene oxidation product is sent to further use.

The process according to the invention is a process for the manufacture of a crude alkane and/or alkene oxidation product starting from a stream of alkane and/or alkene. In a preferred embodiment, the process according to the invention is a process for the manufacture of crude ethylene starting from a stream of methane and/or ethane.

The stream of alkane and/or alkene used in the present invention is a stream containing alkane(s) and/or alkene(s) as main component(s) i.e. a stream which contains more than 50vol% alkane(s) and/or alkene(s), preferably more than 80vol% alkane(s) and/or alkene(s) and even more preferably: more than 90vol% alkane(s) and/or alkene(s). It may also be a stream of pure alkane(s)/alkene(s) i.e. with a purity as available industrially (which does not preclude that impurities may be present, generally in an amount of less than 2vol%, preferably less than 1vol%).The alkane or alkene may be any alkane or alkene having from 1 to 8 carbon atoms, preferably from 1 to 4 carbon atoms. Good results can be obtained with methane and/or ethane, especially with ethane. However, starting from methane (or from a gas comprising methane as main component) is advantageous where such methane is available as natural gas for instance in wells or as shale gas. If necessary, the alkane and/or alkene may be separated from undesired secondary compounds (i.e. impurities which are likely to interfere with the oxidation reaction, induce corrosion etc.) in any known device, for example by absorption, extraction, diffusion or distillation.

It results from the above that the crude alkane and/or alkene oxidation product may also be a mixture of oxidation products.

When starting from ethane which comprises organic compounds containing three carbon atoms (propane, propene), it can be interesting to eliminate those C3 compounds, which can be prejudicial for further use as for example during chlorination/oxychlorination/pyrolysis, and therefore operating with a C2/C3 separation.

This C2/C3 separation can take place either on the stream of ethane before being fed to the catalytic oxydehydrogenation reaction section or on the crude ethylene before its further use, preferably before its use in chlorination/oxychlorination. Preferably, the stream of ethane is subjected to a C2/C3 separation before being fed to the catalytic oxydehydrogenation reaction section.

In the process according to the invention, the stream of alkane and/or alkene is fed to a catalytic oxidation reaction section where at least one exothermic reaction takes place. It may be any exothermic oxidation reaction; it preferably is however an ODH reaction, an OCM reaction or both.

In a preferred embodiment of the invention, the alkane and/or alkene stream comprises ethane, in the oxidation reaction comprises an ODH reaction and in that the oxidation product is ethylene.

In another preferred embodiment, which may be combined with the former one, the alkane and/or alkene stream comprises methane and this methane is first transformed in ethane by an OCM reaction and then, in ethylene by an ODH reaction, said OCM and ODH reactions taking place successively in the reactors of the reaction section.

It is namely so that provided the catalyst (or catalysts since different catalysts can be used in the reactors) is adequately chosen (see below), it may be used for both reactions. Hence, in a preferred embodiment of the invention, the alkane is methane and it is first transformed in ethane by an OCM reaction and then, in ethylene by an ODH reaction, said OCM and ODH reactions taking place successively (and eventually, simultaneously since some methane can still be converted to ethane while some ethane already produced can be converted to ethylene at the same time) in the reactors of the reaction zone. In another preferred embodiment, the alkane is ethane, the oxidation reaction is an ODH reaction and the oxidation product is ethylene.

The terms "catalytic oxydehydrogenation" (referred to as ODH), also known as catalytic oxidative dehydrogenation, are understood to mean a partial oxidation, preferably of ethane, by oxygen in the presence of a catalyst.

The terms "Oxidative Coupling of Methane" (referred to as OCM) are understood to mean a partial oxidation of methane by oxygen in the presence of a catalyst.

When the catalytic reaction is ODH and/or OCM, it generally takes place at a temperature between 300 and 900°C. The ODH/OCM reaction preferably takes place at a temperature of at least 300, preferably of at least 400 and particularly preferably of at least 500°C. The ODH/OCM preferably reaction takes place at a temperature of at most 900, preferably of at most 800, more preferably of at most 750 and most preferably of at most 700°C.

When the catalytic reaction is ODH and/or OCM, it advantageously takes place at a pressure of 1 to 25 bar absolute. The ODH/OCM reaction takes place at a pressure advantageously at least 1, preferably at least 2 and particularly preferably at least 4 bar absolute. The ODH/OCM reaction takes place at a pressure advantageously at most 25, preferably at most 15 and particularly preferably at most 10 bar absolute.

Various catalytic systems may be used to carry out the ODH/OCM reaction according to this embodiment of the invention.

A first group of catalysts are composed of reducible metal oxides, i.e. MoVNb mixed oxides, like the ones detailed in patent application WO 2008/000705 incorporated herein by reference.

A second preferred group of catalysts are the ones supported on alkaline-earth oxides, preferably MgO, which are furthermore advantageously doped with Dy₂O₃. Such catalysts are active both in ODH and OCM reactions and are the object of a co-pending application.

LiCl is advantageously deposited on such supports but catalysts comprising a mixture of different chlorides selected from alkali (sodium, potassium, rubidium or cesium) chlorides and/or earth alkali (strontium or barium) chlorides on such supports are preferred catalysts both for the OCM of methane and for the ODH of ethane.

Particularly preferred catalysts in this second group are
- catalysts comprising mixture of LiCl and NaCl or of LiCl and KCl supported on alkaline-earth oxides, preferably on MgO, more preferably on MgO doped with Dy₂O₃;
- catalysts comprising mixture of LiCl and RbCl or of LiCl and CsCl supported on alkaline-earth oxides, preferably on MgO, more preferably on MgO doped with Dy₂O₃;
- catalysts comprising a mixture of 3 or more different chlorides selected from alkali chlorides and/or earth alkali chlorides supported on alkaline-earth oxides, preferably on MgO, more preferably on MgO doped with Dy₂O₃; and
- catalysts comprising LiCl and one or more chlorides being different from LiCl supported on alkaline-earth oxides, preferably on MgO, doped with a lanthanide oxide different from Dy₂O₃ (preferably samarium oxide, cerium oxide or holmium oxide, more preferably cerium oxide).

In another preferred embodiment of the invention, the alkane and/or alkene stream comprises propane, butane and/or butene.

The reaction section comprises a first reactor and at least one other reactor, all in series. Preferably, the reaction section comprises a first reactor and other reactors all in series. Preferably, these reactors are substantially adiabatic i.e. without voluntary heat exchange to external media (which does not preclude than exchanges may occur owed to the (in)efficiency of the reactors' insulation).

The number of reactors in series is fixed to limit the temperature increase in each reactor, which generally depends on the catalyst used. The temperature increase in each reactor is preferably limited to 100 °C, more preferably to 70°C and even more preferably to 50°C. The reaction section comprises advantageously at least 3 reactors in series. The maximum number of reactors in series is not critical and can be adapted. However, the reaction section comprises more preferably 3 to 4 reactors in series.

Sometimes, it can be advantageous for optimizing the running conditions to have one of the reactors in series which do not serve for the reaction(s) (hence, which is by-passed) but which is in the state of regeneration (i.e. for burning the coke formed during the reaction).

It may also be advantageous to have the different reactors working at different temperatures in order to optimize conversion and selectivity (in which case the temperature may be lower in the last reactor(s)). The same applies to the nature of the catalyst, which may be more active and less selective in the first reactor(s) compared to the last one(s).

The oxygen used in the process of the invention may be oxygen or a gas containing oxygen with other inert gases, such as for example air. Preferably, oxygen is used. The oxygen may or may not be chemically pure. Thus, it is possible to use a very pure source of oxygen containing at least 99 vol% of oxygen but also a source of oxygen containing less than 99 vol% of oxygen. In the latter case, the oxygen used advantageously contains more than 90 vol% and preferably more than 95 vol% of oxygen. A source of oxygen containing from 95 to 99 vol% of oxygen is particularly preferred.

The total amount of oxygen introduced, based on the amount of alkane and/or alkene is advantageously from 0.001 to 2 mol/mol, preferably from 0.005 to 1 mol/mol and particularly preferably from 0.05 to 0.75 mol/mol.

The oxygen stream is preferably divided in as much flows as the number of reactors (i.e. equal amount) before being fed in parallel into each of the reactors in series. Preferably, the oxygen distribution is equal in each of the successive reactors.

The oxygen stream can be preheated or not. Preferably, the oxygen stream is not preheated before being divided into flows and fed into the reactors in series. Preferably also, these flows are not preheated either before being fed in the reactors.

In the process according to the invention, one part of the crude alkane oxidation product leaving the last reactor is recycled as recycled gas to at least one of the reactors in series after having been cooled down while the other part of said crude alkane oxidation product is sent to further use. Besides, the reaction gas is cooled down between each two successive reactors.

The above mentioned cooling of the gas can be obtained using an adequate device like an intercooler (heat exchanger) and/or an ejector, and/or by injection of water either in liquid form (small droplets) or as vapour, the latter being easier but less effective than the former.

When using heat exchanger(s) or water injection, compression is generally required.

When using ejectors, the use of additional heat exchanger(s) and/or compressor(s) may be avoided.

When heat exchangers (intercoolers) are used, these are preferably designed so has to have a small pressure drop in order to reduce the duty of the compressor(s) required to compensate for the pressure drop across the reaction zone. Preferred heat exchangers are of the multi tubular type, of the compabloc type or of the serpentine type.

The complete gas stream leaving the last reactor can be cooled down and afterwards, split in a recycled stream and a stream for further use. Alternatively, the gas stream leaving the last reactor is first split in a recycled stream and a stream for further use, and the recycled stream is then cooled down prior to being recycled. In both cases, a by pass on the cooler may be provided in order to allow/ease process temperature control. As explained above, the crude alkane oxidation product leaving the last reactor and which is recycled as recycled gas contains water which is preferably not eliminated (and even more preferably: not reduced in content) before recycling. Hence, if the further use requires a quenching operation, this quenching will preferably take place entirely after the separation of the gas stream leaving the last reactor into a recycled stream and a further use stream, and on the latter only.

The recycled gas according to the invention preferably contains water in an amount of 10 à 90 vol% and more preferably from 20 to 70 vol%.

According to a first embodiment of the process according to the invention, the recycled gas is recycled to the first reactor of the reactors in series, preferably after compression.

According to such first embodiment, the recycled gas is preferably recycled to the first reactor after having been mixed with the stream of alkane and/or alkene.

More preferably, the recycled gas is recycled to the first reactor after compression and then after having been mixed with the stream of alkane and/or alkene.

According to a first variant of the first embodiment of the process according to the invention, the reaction gas is cooled down between each two successive reactors in intermediate coolers and preferably, to the optimal feeding temperature of the second reactor.

Any kind of cooling fluid can be used in the intermediate coolers.

According to a first alternative, steam generation is used as cooling means in the intermediate coolers so that the cooling fluid is liquid water.

According to a second alternative of such first variant of such first embodiment, the recycled gas is used as cooling fluid in the intermediate coolers.

Therefore, according to this second alternative, advantageously, the recycled gas is recycled to the first reactor of the reactors in series, preferably after compression, after having been then used as cooling fluid in the intermediate coolers and eventually after having been mixed with the stream of alkane and/or alkene.

More preferably, according to this second alternative, the entire stream of crude oxidation product leaving the last reactor is cooled down by an outlet cooler and the temperature of the recycled gas at the inlet of the compression is controlled by a bypass on said outlet cooler.

Most preferably, according to this second alternative, the distribution of the recycled gas in the different intermediate coolers, and eventually the flow of the bypass of the excess of recycled gases, is controlled.

Finally, a possible final adjustment of the feed temperature of the first reactor by the feed heater (the heater heating up the alkane and/or alkene stream) can be considered.

According to a second variant of the first embodiment of the process according to the invention, the reaction gas is cooled down between each two successive reactors by injection of water in such reaction gas.

According to a second embodiment of the process according to the invention, the recycled gas is recycled to each of the reactors in series.

According to such second embodiment, the recycled gas is preferably recycled after compression.

According to such second embodiment, the recycled gas is preferably recycled to the first reactor of the reactors in series after having been mixed with the stream of ethane or not. More preferably, it is recycled to the first reactor after compression and then having been mixed with the stream of alkane and/or alkene.

According to such second embodiment, the recycled gas is advantageously recycled to one of the other reactors than the first one after having been cooled down in an intermediate cooler, preferably after compression, and mixed with the reaction gas leaving the previous reactor before it enters to said reactor.

Therefore, according to such second embodiment, the reaction gas is advantageously cooled down between each two reactors by injection in said reaction gas, part of said recycled gas after having been cooled down in an intermediate cooler, preferably after compression. According to a third embodiment of the process according to the invention, the recycled gas is recycled to the last reactor of the reactors in series and the reaction gas leaving each reactor of the reactors in series is also partly recycled to the reactor which it comes from.

According to such third embodiment, each recycled gas is recycled either after having been cooled down in an intermediate cooler or by addition of water, and afterwards being compressed; or by being sucked by an ejector (the motive fluid of which being advantageously steam) eventually assisted by cooling means (water injection or intermediate cooler).

The latter embodiment (with the ejector) is advantageous since it allows sparing a compressor and since steam is generally available in the process anyway thanks to heat recovery (see above and below). Besides, even the use of a heat exchanger can be avoided.

In the present description, the term "ejector" means the devices, well known to a person skilled in the art, called "steam ejectors". The operation of these devices, which comprise no moving parts, is based on the principle of using a high pressure motive fluid to drive a fluid sucked in at low pressure. The two mixed fluids are discharged at an intermediate pressure. Other details relative to these devices and to the characteristics of their operation can be found, for example, in the catalogue "Jet pumps and gas scrubbers" published in August 1992 by the company GEA WIEGAND Gmbh at W-7505 Ettlingen.

According to such third embodiment, the reaction gas leaving the first reactor can be recycled to the first reactor of the reactors in series after having been mixed with the stream of ethane or not. Preferably, it is recycled to the first reactor after having been mixed with the stream of alkane and/or alkene.

Complementary to the above, a first variant of the third embodiment is such that each recycled gas is cooled down in an intermediate cooler and afterwards compressed, a second variant is such that each recycled gas is cooled down by addition of water and afterwards compressed, a third variant is such that each recycled gas is cooled down in an intermediate cooler and afterwards sucked by an ejector and a fourth variant is such that each recycled gas is sucked by an ejector.

In the process according to the invention, the temperature of the recycled gas is advantageously reduced in a control way in order to provide enough cooling to keep the reactor temperature under control (cooling effect and thermal ballast effect of the recycling gas) and not too much cooling to keep the reaction gas above the dew point (to keep the reaction water in the recycling gas and avoid water condensation) and maintain catalytic activity.

The expression "crude alkane and/or alkene oxidation product" is understood to mean the gas mixture produced by the process starting from a stream of alkane and/or alkene according to the invention. Hence, it is the reaction gas leaving the last reactor. According to the invention, part of this gas is recycled as recycled gas and the other part is sent to further use. Preferably, the recycled gas is not submitted to any treatment before being recycled. More preferably, no water is removed from it. This does not preclude that some secondary constituents might be removed from it before recycling although preferably, the stream is really "crude" i.e. not submitted to any treatment.

When the alkane is ethane, such crude gas mixture contains advantageously ethylene, unconverted ethane, water and secondary constituents. The secondary constituents may be carbon monoxide, carbon dioxide, hydrogen, various oxygen-containing compounds such as, for example, acetic acid or aldehydes, nitrogen, methane, oxygen, optionally acetylene and optionally organic compounds comprising at least 3 carbon atoms.

When the alkane is methane, such crude gas mixture contains advantageously ethylene, ethane, unconverted methane, water and secondary constituents. The secondary constituents may be carbon monoxide, carbon dioxide, hydrogen, various oxygen-containing compounds such as, for example, acetic acid or aldehydes, nitrogen, methane, oxygen, optionally acetylene and optionally organic compounds comprising at least 3 carbon atoms.

Preferably, the stream of alkane and/or alkene is preheated before being fed to the first reactor of the reaction section and the therefore, the reaction section advantageously comprises a startup feed heater in which the feed of alkane and/or alkene is preheated before being fed to the first reactor.

When the stream of alkane and/or alkene is preheated and the recycled gas is mixed with the stream of alkane and/or alkene, the most favourable situation is to control the temperature of the recycling gas in order to reach the first reactor feeding temperature without any addition of heat in the feed heater.

When the alkane is methane and/or ethane, the crude ethylene obtained by the process according to the invention can be used in several different processes in view of manufacturing organic chemical compounds, among which 1,2-dichloroethane, which hence corresponds to a further use according to the invention. Other uses may be the manufacture of ethylene derivatives like ethylene oxide and/or its derivatives, ethylene glycol and/or its derivatives, ethylene amines, and/or polyethylene

Therefore, according to a preferred embodiment of the present invention, the other part of said crude ethylene sent to further use is advantageously fed to a quench section in order to separate water there from and is afterwards compressed and optionally submitted to further purification before being conveyed to chlorination/oxychlorination in order to obtain 1,2-dichloroethane which is preferably subjected to a pyrolysis in order to produce vinyl chloride which is preferably polymerized to produce polyvinyl chloride.

In the quench section, the other part of said crude ethylene sent to further use is advantageously cooled down and water condensed and separated.

Before being fed to the quench section, the other part of said crude ethylene sent to further use can be submitted to a heat recovery or not. Preferably, the other part of said crude ethylene sent to further use is submitted to a heat recovery before being fed to the quench section.

Therefore, according to the process according to the invention, the other part of said crude ethylene sent to further use is advantageously fed, after heat recovery, to a quench section in order to separate water there from and is afterwards compressed and optionally submitted to further purification before being conveyed to chlorination/oxychlorination in order to obtain 1,2-dichloroethane which is preferably subjected to a pyrolysis in order to produce vinyl chloride which is preferably polymerized to produce polyvinyl chloride.

After compression, the other part of said crude ethylene sent to further use can be used directly if its composition is suitable for chlorination/oxychlorination. If not, it is preferably submitted to further purification before being conveyed to chlorination/oxychlorination.

Further purification may comprises the drying and/or other additional treatments in order to remove any compounds that are not desired in the chlorination/oxychlorination of the other part of said crude ethylene sent to further use.

Another object of the present invention is a process for the manufacture of DCE using the other part of crude ethylene sent to further use obtained by the process for the manufacture of crude ethylene starting from a stream of methane and/or ethane according to the invention.

The DCE obtained by chlorination/oxychlorination of ethylene is then preferably subjected to a pyrolysis in order to produce vinyl chloride (VC) which is preferably in turn polymerized to produce polyvinyl chloride (PVC).

Another advantage of the process according to the invention is that it makes it possible to have, on the same industrial site, a completely integrated process ranging from the hydrocarbon source - namely methane and/or ethane - up to the polymer (PVC or polyethylene) obtained starting from the monomer manufactured.

Some embodiments, variants and alternatives of the process according to the invention are illustrated in Figures 1 to 7.
- Figure 1 illustrates the first alternative of the first variant of the first embodiment of the process according to the invention.
- Figure 2 illustrates the second alternative of the first variant of the first embodiment of the process according to the invention.
- Figure 3 illustrates the second embodiment of the process according to the invention.
- Figure 4 illustrates the first variant of the third embodiment of the process according to the invention.
- Figure 5 illustrates the second variant of the third embodiment of the process according to the invention.
- Figure 6 illustrates the second variant of the first embodiment of the process according to the invention.
- Figure 7 illustrates the third variant of the third embodiment of the process according to the invention.

In these Figures, identical numbers tend to designate identical items, namely:
1: feed heater
2: reactor
3: intercooler
4: by-pass
5: compressor
6: ejector

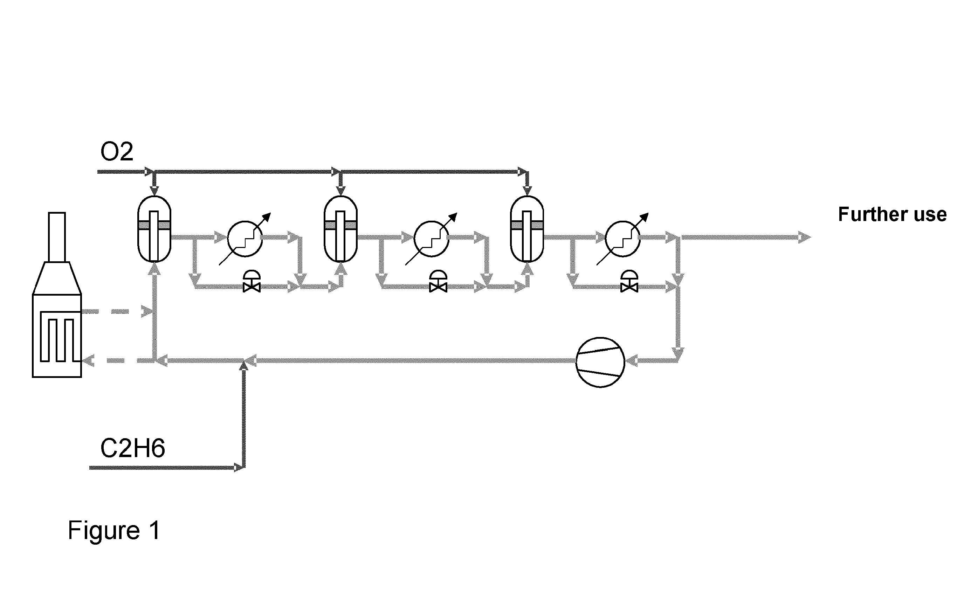

The embodiment of Figure 1 implies recycling the recycled gas leaving the last reactor to the first one. Before said recycled gas enters said first reactor, it is:
- cooled down thanks to a heat exchanger (intercooler generating steam i.e. steam boiler) which also cools down the stream for further use but can be by passed in order to control the temperature of said recycled gas and keep it at the required level
- compressed in order to compensate for the pressure drop within the reactors and the intercoolers
- mixed with the stream of alkane and/or alkene
- the mixture of both is eventually heated in a feed heater; as explained above, this can eventually be prevented by adapting the temperature of the recycled gas, provided the flow rates allow to do so. This feed heater can also be used for start up conditions.

The gas stream leaving each reactor is actually cooled down in the same way as the recycled gas i.e. using a steam boiler provided with a by pass.

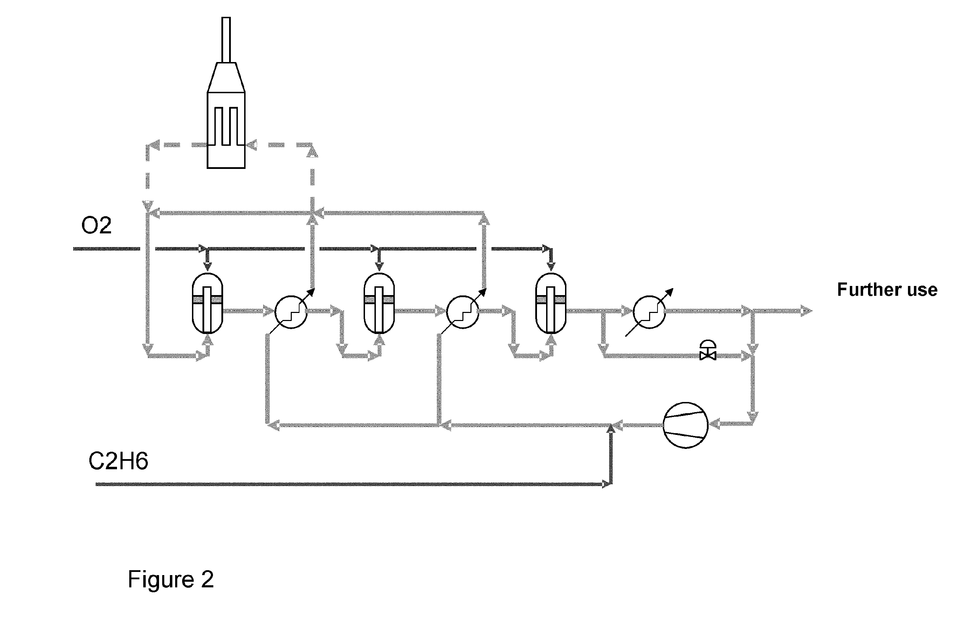

The embodiment of Figure 2 is close to the one of Figure 1 but instead of using steam boilers to cool down the reaction gas between each two successive reactors, it uses heat exchangers where the cooling fluid is a partial flow of the recycled gas. The partial flows of recycled gas leaving each of these heat exchangers are recycled to the first reactor.

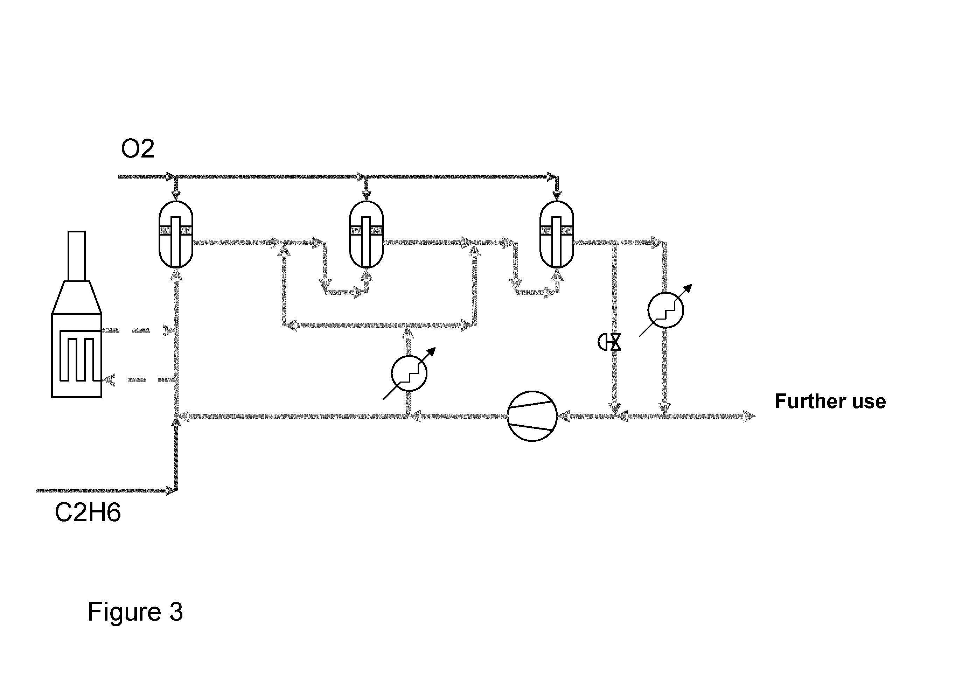

The embodiment of Figure 3 is close to the one of Figures 1 and 2 but instead of using heat exchangers to cool down the reaction gas between each two successive reactors, it uses the injection of a partial flow of the recycled gas in between them, said partial flow having been cooled down first.

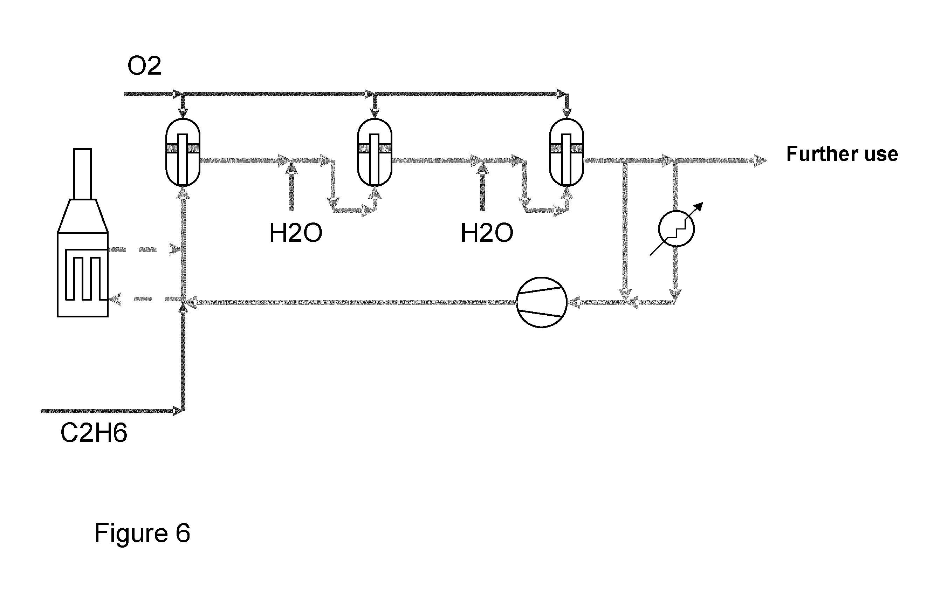

The embodiment Figure 6 is again close to the one of Figures 1 and 2 but instead of using heat exchangers to cool down the reaction gas between each two successive reactors, it uses the injection of water in between them.

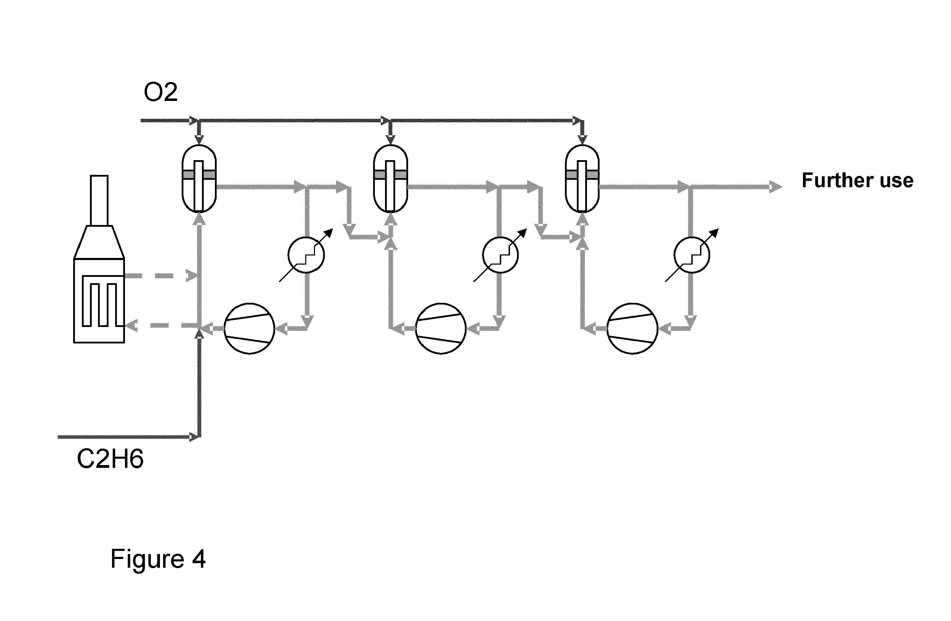

In the embodiments of Figures 4, 5 and 7, the reaction gas leaving each reactor is partly recycled to the reactor which it comes from, after having been cooled. In the embodiment of:
- Figure 4, this cooling is performed using a steam boiler as in Figure 1, followed by a compression to compensate for the pressure drop

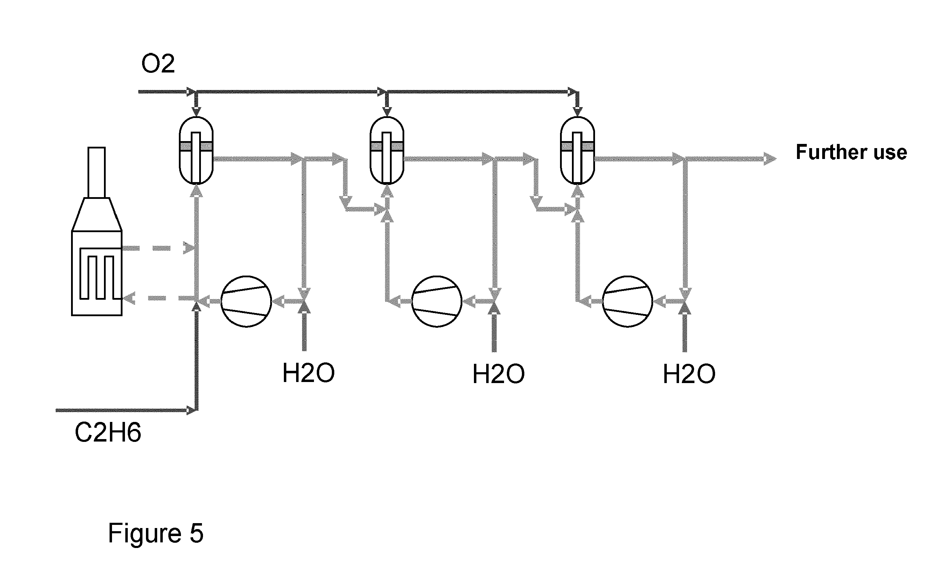

- Figure 5, this cooling is performed by injection of water, followed by a compression to compensate for the pressure drop

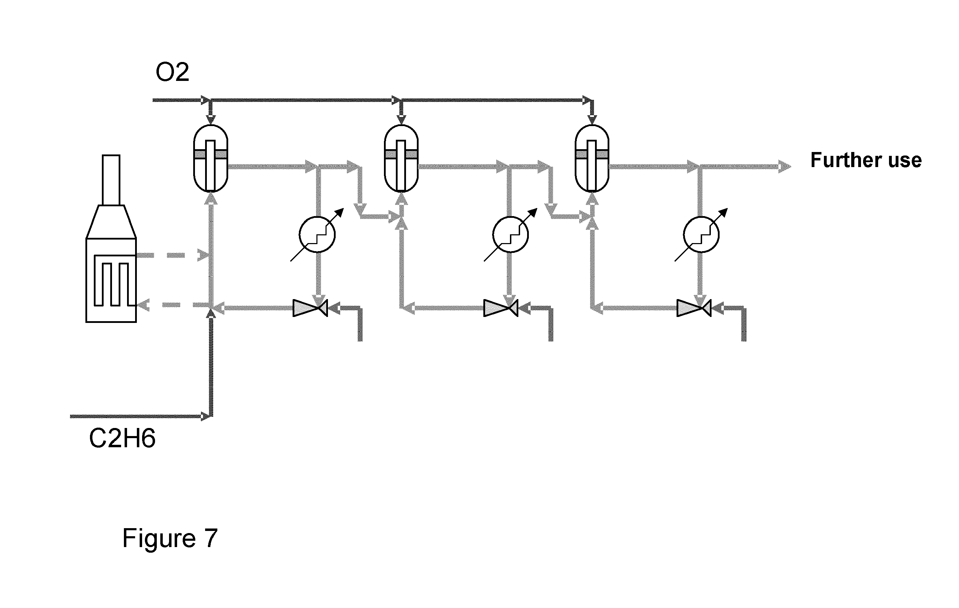

- Figure 7, this cooling is performed using a steam boiler as in Figure 1, followed by an injector; it is worth noting that provided the fluid powering the injector is appropriate in nature, flow and temperature, the recourse to an heat exchanger can be avoided.

## Claims

1. Process for the manufacture of a crude alkane and/or alkene oxidation product according to which a stream of alkane and/or alkene and a stream of oxygen are fed to a catalytic oxidation reaction section comprising a first reactor and at least one other reactor, all in series, in which
- the stream of alkane and/or alkene is fed into the first reactor for catalytic oxidation with an oxygen flow in order to produce a reaction gas which is then fed successively to the following reactors in series for further catalytic oxidation;
- the stream of oxygen is divided in several oxygen flows which are fed in parallel into each of the reactors in series;
- the reaction gas is cooled down between each two successive reactors and finally produces a crude alkane and/or alkene oxidation product leaving the last reactor;
- one part of said crude alkane and/or alkene oxidation product leaving the last reactor is cooled down and recycled as recycled gas to at least one of the reactors in series while the other part of said crude alkane and/or alkene oxidation product is sent to further use.

2. Process according to Claim 1, **characterized in that** the alkane and/or alkene stream comprises ethane, **in that** the oxidation reaction comrpises an ODH reaction and **in that** the oxidation product is ethylene.

3. Process according to Claim 1 or 2, **characterized in that** the alkane and/or alkene stream comprises methane and **in that** this methane is first transformed in ethane by an OCM reaction and then, in ethylene by an ODH reaction, said OCM and ODH reactions taking place successively in the reactors of the reaction section.

4. Process according to either Claim 2 or 3, **characterized in that** the catalytic oxidation reaction(s) take place at a temperature between 300 and 900°C.

5. Process according to either of Claims 2 to 4, **characterized in that** the catalytic oxidation reaction(s) take place at a pressure of 1 to 25 bar.

6. Process according to Claim 1, **characterized in that** the alkane and/or alkene stream comprises propane, butane and/or butene.

7. Process according to any one of Claims 1 to 6, **characterized in that** the recycled gas is recycled to the first reactor of the reactors in series.

8. Process according to Claim 7, **characterized in that** the reaction gas is cooled down between each two successive reactors in intermediate coolers.

9. Process according to Claim 8, **characterized in that** steam generation is used as cooling means in the intermediate coolers.

10. Process according to Claim 8, **characterized in that** the recycled gas is used as cooling fluid in the intermediate coolers.

11. Process according to Claim 7, **characterized in that** the reaction gas is cooled down between each two successive reactors by injection of water in such reaction gas.

12. Process according to any one of Claims 1 to 6, **characterized in that** the recycled gas is recycled to each of the reactors in series.

13. Process according to any one of Claims 1 to 6, **characterized in that** the recycled gas is recycled to the last reactor of the reactors in series and **in that** the reaction gas leaving each reactor of the reactors in series is also partly recycled to the reactor from which it comes.

14. Process according to Claim 13, **characterized in that** each recycled gas is recycled either after having been cooled down in an intermediate cooler or by addition of water, and afterwards being compressed; or by being sucked by an ejector.

15. Process according to any of Claims 2 to 5, **characterized in that** the other part of said crude ethylene being sent to further use is fed, preferably after heat recovery, to a quench section in order to separate water there from and is afterwards compressed and optionally submitted to further purification before being conveyed to chlorination/oxychlorination in order to obtain 1,2-dichloroethane which is preferably subjected to a pyrolysis in order to produce vinyl chloride which is preferably polymerized to produce polyvinyl chloride.
